# EUROPEAN PATENT SPECIFICATION

(11) **EP 4 308 026 B1**
(45) Date of publication and mention of the grant of the patent: **10.12.2025**
(21) Application number: 22715275.8
(22) Date of filing: 17.03.2022
(51) Int. Cl.: A61B 18/22, A61B 18/24

(54) **FEMTOSECOND LASER DEVICE FOR MINIMALLY-INVASIVE SURGERY**
FEMTOSEKUNDENLASERVORRICHTUNG FÜR MINIMALINVASIVE CHIRURGIE
DISPOSITIF LASER À FEMTOSECONDE POUR CHIRURGIE MINIMALEMENT INVASIVE

(30) Priority: 17.03.2021 US 202163162317 P
(43) Date of publication of application: 24.01.2024
(73) Proprietor: Okinawa Institute of Science and Technology School Corporation, Kunigami-gun, Okinawa 904-0495 (JP)
(72) Inventor: LISICOVAS, Viktoras, Kunigami Okinawa 904-0495 (JP); DANI, Keshav M., Kunigami Okinawa 904-0495 (JP); MADÉO, Julien, Kunigami Okinawa 904-0495 (JP); SASTRY, Ashwani, Kunigami Okinawa 904-0495 (JP)
(74) Representative: Scott, Stephen John
(86) International application number: PCT/US2022/020810
(87) International publication number: WO 2022/197965

(56) References cited:
- US-A- 6 156 030
- US-A1- 2006 056 468

## Description

### TECHNICAL FIELD

The present application relates to laser surgical devices.

### BACKGROUND

Existing devices for atherectomy clear plaque generally in two ways: (i) by removing material through mechanical action; and (ii) removing material by breaking molecular bonds via high-energy ultraviolet light from a laser source. Laser removal-based approaches are preferable since removal of unwanted material by forceful mechanical action in close proximity to healthy tissue carries significant risks, e.g., perforation of blood vessels.

Current state-of-the-art excimer laser-based devices employ nanosecond pulses that perform material removal in the linear absorption regime. However, efficient removal in the linear absorption regime is sensitive to the material's absorptive properties and is also of limited use in processing blockage with high mineral content. In an attempt to address this issue hybrid systems have been recently introduced using both laser and mechanical removal of the material.

In contrast, in the case of femtosecond lasers, the compression of light energy into ultrashort pulses (~10^-12 - 10^-15 seconds) results in peak power densities that are sufficient to ionize atoms resulting in an optical breakdown of the material (further referred to as ablation). In this context, document US6156030 is mentioned.

The timescales are too short for thermal transport, which eliminates local heat accumulation and corresponding thermal damage to nearby tissue and blood vessels. Furthermore, the ablation process is much less dependent on tissue absorption properties. As such, the device can employ near-infrared light for ablation - a wavelength range that is inherently less harmful and thus minimizes peripheral damage to living tissue due to stray light exposure. In contrast, ultraviolet radiation employed in the nanosecond pulsed excimer lasers in use for atherectomy currently, has the potential to cause damage on the cellular level. Finally, femtosecond pulsed light offers the improved cut precision and absence of fractures due to operation in the non-linear high peak power density regime.

Internal surgery devices for surface microsurgery with ultrashort pulses (~1ps) have been attempted with some success by several research groups. One approach uses a piezo cantilever resonator to precisely guide a focused laser spot over a target. Another approach uses a conventional multi-core fiber bundle to deliver light energy in chirped pulses and then refocus the pulses by wavefront shaping to achieve ablation. Both approaches, while useful for surface microsurgery, only allow for a small field-of-view to be addressed compared to device dimensions and therefore are not compatible with atherectomy.

Despite the advantages of ultrashort pulse ablation, femtosecond based atherectomy has not been possible due to the lack of means to deliver and control high energy femtosecond pulses inside the body.

### SUMMARY

The invention is defined by the appended set of claims.

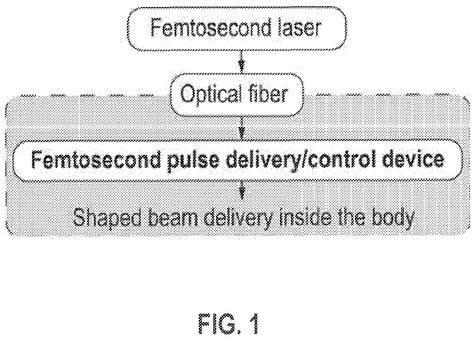

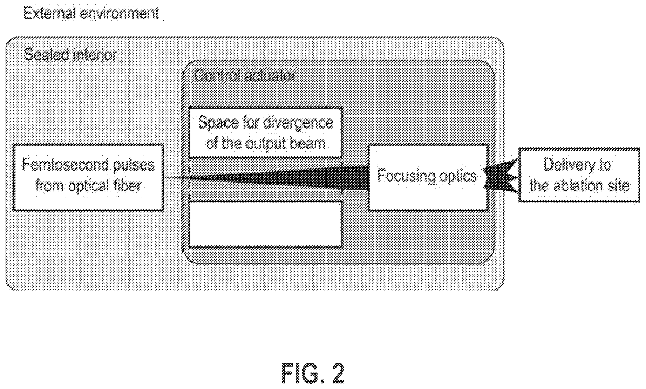

A femtosecond laser device for minimally-invasive surgery includes an ultrafast (femtosecond or sub-100 picosecond) pulsed light source coupled with a flexible optical fiber capable of transmission of femtosecond or picosecond pulses. The optical fiber carries light to the location of surgery inside the body. An implementation includes an endcap for the fiber that transmits the femtosecond light from the laser to the location of the surgery (Fig. 1). The fiber optical component and the endcap are capable of handling pulse energies and durations necessary to achieve a target peak power density of at least 1.5 TW/cm² over the area of the focus to ensure the ablation of both organic and inorganic material. To address the challenge of the optical breakdown damage threshold of component pieces manipulating the femtosecond pulses, an implementation includes an internal free space to allow for the divergence of the laser beam propagating from the optical fiber. The divergence distance is governed by the numerical aperture of the fiber. The distance is chosen such that the incoming laser beam fills the back aperture of the focusing optics positioned on the optical path and does not exceed the damage threshold of these components. Focusing optics are then used to achieve focused regions of higher intensity appropriate for the ablation of the target material. A variety of focusing optics, including refractive, reflective, diffractive, or a combination can be used to achieve a focus shape tailored for the specific application, such as line, spiral, point cloud or arbitrary pattern tailored for the specific application. These shapes can be achieved by one or a combination of light focusing optical elements: for example, cylindrical or aspherical lenses, curved mirrors, diffraction gratings, holographic diffraction gratings, phase masks or optical meta surfaces. These focusing optics are fixed on an actuator positioned in a sealed external shell. The actuator moves the optical element or elements relative to the output beam by translation or rotation of optical components, independently of the external shell or through a rotation of the whole device. This allows the high intensity focused femtosecond pulses to be delivered to the site of surgery and controlled to achieve ablation intensities. The design also prevents damage to optics and maintains a separation between internal components and the external environment (Fig. 2).

### BRIEF DESCRIPTION OF THE DRAWINGS

Fig. 1 depicts a system to utilize a femtosecond device for transmission and control of femtosecond pulses for atherectomy and other internal surgery applications.
Fig. 2 depicts a general operating principle of the femtosecond device for transmission and control of femtosecond pulses.
Fig. 3 depicts a high-level overview of components and design of the device and supporting elements for atherectomy and other internal surgery applications.
Fig. 4 depicts components of a first implementation for atherectomy and other internal surgery applications generating a forward-facing light sheet for ablation.
Fig. 5 depicts light propagation and control of the forward-facing light-sheet in the first implementation.
Fig. 6 depicts components of a second implementation for atherectomy and other internal surgery applications by generating a lateral light sheet.
Fig. 7 depicts light propagation and control of the lateral light-sheet in the second implementation.
Fig. 8 depicts potential application of the first implementation, the second implementation and a fourth implementation in atherectomy and related surgical procedures.

### OVERVIEW

In an implementation, a system comprises an ultrafast laser configured to generate light pulses of less than 100 picoseconds, an optical fiber configured to transmit the light pulses from the ultrafast laser, an optical fiber endcap device comprising one or more optical elements configured to lower and then raise the intensity of the light pulses from the optical fiber and create an arbitrary optical pattern from the light pulses of the optical fiber. The one or more optical elements are configured to focus an incoming beam from the optical fiber to a peak power density of greater than 10¹² W/cm².

In an implementation, a method of using a femtosecond laser ablation device to perform a laser ablation procedure comprises inserting an optical fiber and ablation device into a blood vessel, generating, through a femtosecond laser, a plurality light pulses of less than 100 picosecond with pulse energies between 10⁻⁷ J and 10⁻² J, wherein the ablation device comprises one or more optical elements configured to focus an incoming beam to a peak power density of greater than 10¹² W/cm², and focusing an output of the laser ablation device onto an ablation site in the blood vessel.

In another implementation, a method of using a femtosecond laser ablation device to perform a laser ablation procedure comprises inserting an optical fiber and ablation device into a body of a person, generating, through a femtosecond laser, a plurality light pulses of less than 100 picosecond with pulse energies between 10⁻⁷ J and 10⁻² J. The ablation device comprises a focusing optics assembly configured to focus an incoming beam to a peak power density of greater than 10¹² W/cm², and focus an output of the ablation device onto an ablation site in the body for ablation of body tissue or foreign material. Example applications include, without limitation: removal of biological tissue or foreign material within the gastrointestinal tract; removal of cartilage, bone, ligament, or foreign material within a joint space or vertebral space within the body; removal of thrombotic material within the venous system, the heart, or the pulmonary arteries; removal of inter-atrial septum to allow passage of equipment from the right to the left side of the heart; removal of hypertrophied cardiac tissue of a heart with hypertrophic cardiomyopathy; removal of biological tissue or a foreign body within the abdominal cavity or retroperitoneal space; or removal of biological tissue or a foreign body within the urethra, bladder, ureters, or renal calyces by navigating the device via cystoscopy or direct puncture of the renal calyces, and placing the device adjacent to the material to be ablated using fluoroscopic, fiber-optic, or ultrasound guidance.

The described implementations enable the use of femtosecond pulses for bulk removal of tissue (surgery) or other materials (e.g., calcified plaque) inside the body, in particular inside a blood vessel such as an artery. One target application is atherectomy to treat the narrowing of blood vessels caused by the accumulation of organic/mineral blockage. The implementations can also be used for other outpatient applications that benefit from removal of bulk material inside the body, such as kidney stone removal, removal of tumors or cancerous cells, removal of thrombus (clot) from the venous system, extraction of foreign bodies in the vascular system (laser lead extraction), or other parts of the body, removal of bone or cartilage within joint spaces within the body.

### A Femtosecond Laser Device

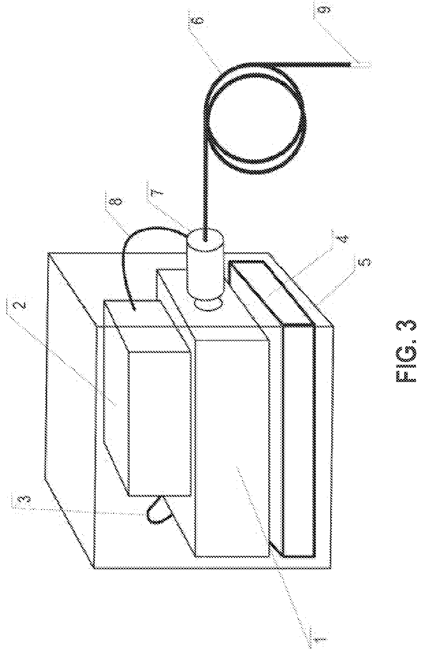

In an implementation, with reference to Fig. 3, a device comprises a femtosecond laser source (1), a hardware controller and power supply (2), interfacing with the femtosecond laser source (1) via a control cable (3). These components can be placed on a vibration isolation system (4) for protection from external vibration and enclosed with light enclosure (5) to prevent light leakage. Laser light is injected into the optical fiber (6) through a fiber coupler (7). The optical fiber (6) can be a single fiber or a fiber bundle. Additionally, the control and power supply wiring (8) may interface with the optical fiber (6) through a fiber coupler (7). The apparatus terminates in a femtosecond ablation device (9).

A first implementation generates a forward-facing light sheet focus relative to the device. A second implementation generates a light sheet focus facing perpendicular to the device. A third implementation combines aspects of the first and second implementations to provide both a forward light sheet and a perpendicular light-sheet either simultaneously or by switching between two modes on-demand. A fourth implementation is an extension of the first implementation.

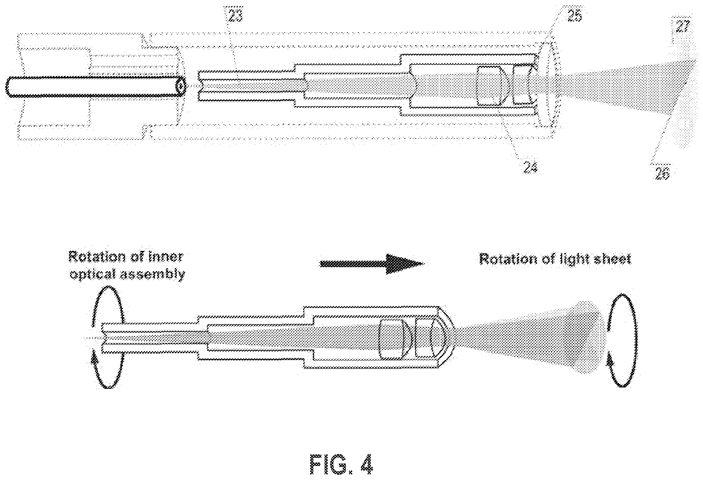

Figs. 4-7 depict example ablation devices according to one or more implementations. The ablation device (9) includes a flexible protective biocompatible shell (10) that is made of a biocompatible material enveloping flexible protective internals (11) for the fiber core and cladding (12), such as a flexible mono coil. The flexible portion of the fiber composed of (10), (11), (12) is connected to the ablation device (9) through an airtight connector (13) sealed with glue or by other means. Control/power circuitry (14) is embedded in (10), (11), or (12), and passed through the airtight connection (13), or provided separately as an external wire. The control circuitry is provided to a power and control actuator or actuators (15) for changing the position, bending angle, and rotation angle of the internal rotor (16). The actuator or actuators (15) here may be implemented using an ultrasonic piezo actuator due to requirement for small size, but can also be implemented by a motor based on piezoelectric, electrostatic, electromagnetic, electro-conjugate fluid, thermal mode of operation, bimetal plates, inductive actuator, or any other suitably sized actuator component. The motion of the rotor (16) is stabilized by a suitable bearing (18), such as a ball bearing or based on a self-lubricating plastic. To provide stable rotation and minimize vibrations, a counterweight (19) may be included on the distal end of the rotor (16). The focusing optics assembly (20) is attached to the rotor (16). The design may be enclosed in a rigid, air-tight shell (21) containing a transparent exit window (22) ensuring that the movement of internal components does not interfere with the external environment (Fig. 4).

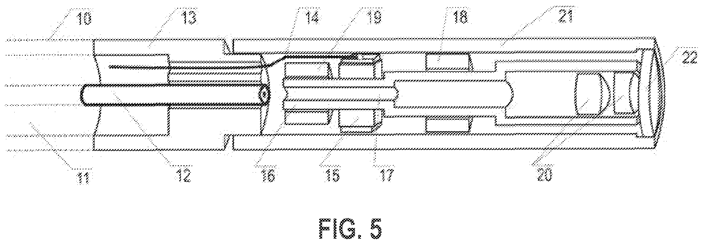

Fig. 4 depicts components of the first implementation for atherectomy and other internal surgery applications generating a forward-facing light sheet for ablation. The forward-facing light-sheet design contains two focusing elements to manipulate the diverging beam propagating through a spacer (23). A cylindrical lens focuses down the incoming laser beam to a line (24) in one direction and a second cylindrical lens expands the beam in the perpendicular direction (25) (Fig. 4). This results in a forward-facing light sheet (26) that rotates by the action of the actuator around the central axis and scans the ablation plane (27) projecting a light sheet of sufficiently high-intensity light to achieve ablation. In this implementation focusing optics are chosen such as to produce ablation over the full diameter of the device, enabling drilling through the target material (Fig. 5).

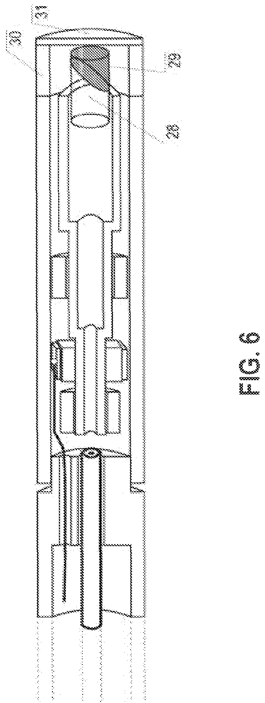

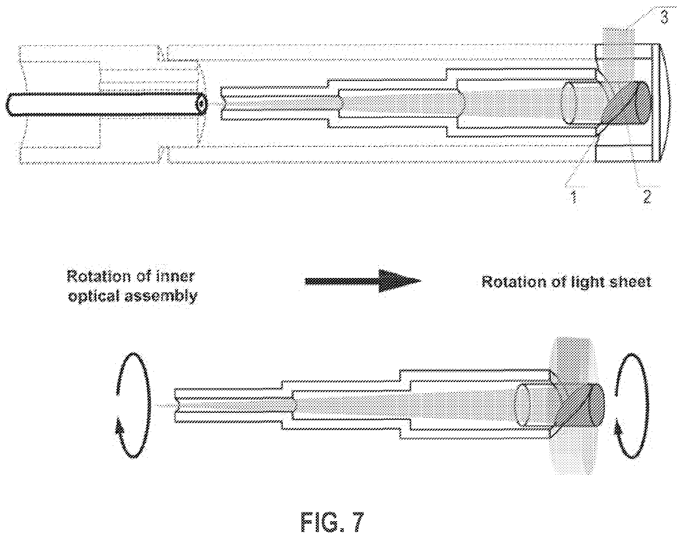

Fig. 6 depicts components of the second implementation for atherectomy and other internal surgery applications generating a lateral light sheet. In the second implementation, the forward-facing light sheet design to generate a perpendicular light sheet has the same basic structure as previously described with the exceptions of the focusing assembly and the exit window. In this implementation, the optical assembly is comprised of a transparent optical cylinder (28), intersected diagonally by a mirror (29). An alternative to using the mirror (29) includes selecting the refractive index of the material for the transparent optical cylinder (28) such that total internal reflection takes place at the diagonal cut. The end of the device is capped by a transparent tube (30). The distal cap (31) may be made of non-transparent material in case of the perpendicular line-focus design only (Fig. 6). In this implementation, the divergent beam from the optical fiber enters the cylindrical optic from the top and is reflected in a perpendicular direction (32). The side of this cylinder (33) acts as a cylindrical lens to focus light into a line parallel to the device (33), where intensity is sufficiently high to achieve ablation. The rotation of the inner assembly around the central axis moves this line focus around the device (Fig. 7) enabling the ablation of material on the periphery of the device.

A third implementation combines the optical elements of perpendicular focusing design and forward-focusing design, that simultaneously ablate in front of the device and perpendicularly. In this implementation, the mirror (29) is replaced by a beam splitter for simultaneous ablation in both forward and lateral directions. Alternatively, switching between the two modes may be implemented by replacing the mirror (29) with a device capable of controlled reflectivity, for instance using an electrically switchable transflective mirror based on a liquid crystal layer.

In a fourth implementation, the forward-focusing design of the first implementation is embedded in the side of a flexible hollow shell. The shell has a central space to capture foreign bodies. The device is gradually rotated around the object to remove the obstruction surrounding it.

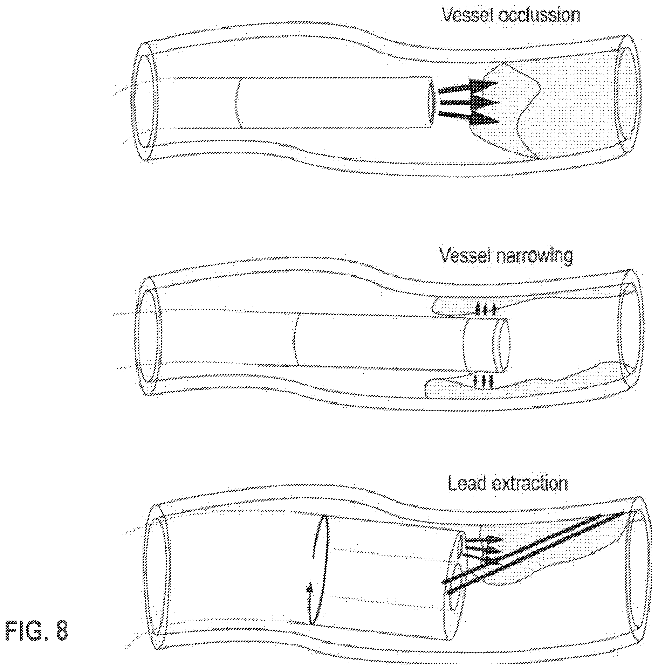

The first implementation is designed to address complete or partial occlusion in a blood vessel, where the device diameter is too large to pass through the blockage. The second implementation is designed to remove a blockage, where narrowing of blood vessels is caused by the lateral accumulation of blockage but is not so severe as to prevent the device from passing through (Fig. 8). A potential application is to use the first implementation and the second implementation in sequence to clear larger occlusions by first introducing a channel through the occlusion and then progressively widening it.

The third implementation enables both types of procedures without the need to exchange the devices. The fourth implementation is useful for removal of surgical leads after cardiac surgery. The proposed devices are intended for application to atherectomy but can be used in other medical procedures, for example, removal of kidney stones, ablation of bone or cartilage within a joint space or spinal space, or other surgical interventions where material with high mineral content or high density poses a problem. Another potential application includes removal of foreign bodies by capturing and ablating vaporizing the foreign body, eliminating the need for open surgical removal.

In an implementation, the system comprises a fiber coupling device capable of transmitting light pulses of less than 100 picosecond in infrared, near-infrared, visible, or ultraviolet spectrum with pulse energies between 10⁻⁷ J and 10⁻² J.

In an implementation, the fiber coupling device is configured to be capable of holding elevated internal gas pressure, or capable of internal vacuum.

In an implementation, the system comprises an optical fiber, capable of transmitting light pulses of less than 100 picosecond in infrared, near-infrared, visible, or ultraviolet spectrum with pulse energies between 10⁻⁷ J and 10⁻² J.

In an implementation, the optical fiber comprises a bend radius of at least 30 cm and length of at least 1 m.

In an implementation, the system comprises an endcap enclosure that is hermetically sealed, capable of holding elevated internal gas pressure, or capable of internal vacuum.

In an implementation, the system comprises an internal spacer to allow for divergence of the beam outputted by the fiber to a peak power density below 10¹² W/cm².

In an implementation, the system comprises an optical device or assembly that can down-focus the incoming beam to a peak power density above 10¹² W/cm².

In an implementation, the optical device or assembly is configured to generate arbitrary shaped focus (other than point focus) to accelerate scanning.

In an implementation, the system comprises an actuator configured to move or rotate the optical assembly in relation to the fiber to achieve scanning implemented using an ultrasonic piezo actuator or a motor based on piezoelectric, electrostatic, electromagnetic, electro-conjugate fluid, thermal mode of operation, bimetal plates, inductive actuator.

In an implementation, the system utilizes a second lumen adjacent but conjoined to the fiber / end cap that allows passage of a guidewire to guide position and trajectory of the device. The guidewire configuration may be a rapid exchange or an over the wire configuration.

In an implementation, the device may be passed through an endoscope, colonoscope, or esophagogastroduodenoscope to allow ablation of biological material or a foreign body within the GI tract.

In an implementation, the device may be passed through an arthroscope to allow ablation of biological material, bone, cartilage, or a foreign body within a joint space or vertebral space.

In an implementation, the device may be passed through the venous system, right sided chambers of the heart, or the pulmonary arteries to ablate acute or chronic thrombus within the venous system (thrombolectomy).

In an implementation, the device may be guided into position and placed against the atrial septum of the heart to allow creation of a channel between the right atrium and the left atrium (trans-septal puncture).

In an implementation, the device may be navigated against the hypertrophic septum of a heart with hypertrophic cardiomyopathy as a safe alternative to alcohol septal ablation.

In an implementation, the housing of the device may also incorporate a second lumen to allow passage of an intravascular ultrasound probe, to facilitate guidance and positioning of the ablation tip.

In an implementation, the ablation tip may be positioned in the sub-intimal space of a chronic occlusion, adjacent to the true lumen, and the subintimal tissue may be ablated to allow creation of a channel into the distal true lumen of an occluded vessel.

In an implementation, the device may incorporate a nitinol endovascular snare to capture a foreign body or biological material within the body.

In an implementation, the device may be passed into the peritoneal cavity or retroperitoneal cavity via a laparoscopic approach to allow ablation of biological material or a foreign body within the abdominal cavity.

In an implementation, the device may be passed into the body within the urethra, bladder, ureters, or renal calyces by navigating the device via cystoscopy or direct puncture of the renal calyces and used to ablate biological tissue or foreign material within the any of the aforementioned structures.

Any of the implementations above may include a suction / negative pressure / vacuum system to capture and remove any debris created in the ablation process.

### Example Use Cases

In an implementation, a method of using a femtosecond laser ablation device to perform atherectomy / thrombectomy / ablation of foreign material, comprises inserting, generating, focusing. In an implementation, an optical fiber and ablation device is inserted into a blood vessel. In an implementation, the light pulses are of less than 100 picoseconds. In an implementation, the light pulses have energies between 10⁻⁷ J and 10⁻² J. In an implementation, the ablation device comprises a focusing optics assembly configured to focus an incoming beam to a peak power density of greater than 10¹² W/cm². In an implementation, an output of the ablation device is focused onto an ablation site in the blood vessel.

In an implementation, a method of using a femtosecond laser ablation device to ablate biological tissue or foreign material within the gastrointestinal tract, comprises inserting, generating, focusing. In an implementation, an optical fiber and ablation device is inserted into the GI tract through an esophagogastroduodenoscope or colonoscope and placing the fiber adjacent to the biological tissue or foreign material to be ablated. In an implementation, the light pulses are of less than 100 picoseconds. In an implementation, the light pulses have energies between 10⁻⁷ J and 10⁻² J. In an implementation, the ablation device comprises a focusing optics assembly configured to focus an incoming beam to a peak power density of greater than 10¹² W/cm². In an implementation, an output of the ablation device is focused onto an ablation site in the GI tract.

In an implementation, a method of using a femtosecond laser ablation device to ablate cartilage, bone, ligament, or foreign material within a joint space or vertebral space within the body, comprises inserting, generating, focusing. In an implementation, an optical fiber and ablation device is inserted into a joint space under open or fiber-optic visualization (arthroscopy) and placing the fiber adjacent to the material to be ablated. In an implementation, the light pulses are of less than 100 picoseconds. In an implementation, the light pulses have energies between 10⁻⁷ J and 10⁻² J. In an implementation, the ablation device comprises a focusing optics assembly configured to focus an incoming beam to a peak power density of greater than 10¹² W/cm². In an implementation, an output of the ablation device is focused onto an ablation site in the joint space.

In an implementation, a method of using a femtosecond laser ablation device to ablate thrombotic material within the venous system, the heart, or the pulmonary arteries comprises inserting, generating, focusing. In an implementation, an optical fiber and ablation device is inserted into a vein in the body and potentially into the right atrium, right ventricle, or pulmonary artery and ablating thrombotic material within these structures. In an implementation, the light pulses are of less than 100 picoseconds. In an implementation, the light pulses have energies between 10⁻⁷ J and 10⁻² J. In an implementation, the ablation device comprises a focusing optics assembly configured to focus an incoming beam to a peak power density of greater than 10¹² W/cm². In an implementation, an output of the ablation device is focused onto thrombotic material in the aforementioned structures.

In an implementation, a method of using a femtosecond laser ablation device to ablate the inter-atrial septum to allow passage of equipment from the right to the left side of the heart comprises inserting, generating, focusing. In an implementation, an optical fiber and ablation device is inserted into a vein in the body and placing the ablation tip adjacent to the inter-atrial septum using fluoroscopic and / or intracardiac echo guidance. In an implementation, the light pulses are of less than 100 picoseconds. In an implementation, the light pulses have energies between 10⁻⁷ J and 10⁻² J. In an implementation, the ablation device comprises a focusing optics assembly configured to focus an incoming beam to a peak power density of greater than 10¹² W/cm². In an implementation, an output of the ablation device is focused onto the inter-atrial septum to create a channel between the right and left ventricles.

In an implementation, a method of using a femtosecond laser ablation device to ablate hypertrophied cardiac tissue of a heart with hypertrophic cardiomyopathy comprises inserting, generating, focusing. In an implementation, an optical fiber and ablation device is inserted into an artery in the body and placing the ablation tip adjacent to the inter-atrial septum using fluoroscopic and / or intracardiac echo guidance. In an implementation, the light pulses are of less than 100 picoseconds. In an implementation, the light pulses have energies between 10⁻⁷ J and 10⁻² J. In an implementation, the ablation device comprises a focusing optics assembly configured to focus an incoming beam to a peak power density of greater than 10¹² W/cm². In an implementation, an output of the ablation device is focused onto hypertrophied cardiac tissue to ablate this tissue.

In an implementation, a method of using a femtosecond laser ablation device to ablate biological tissue or a foreign body within the abdominal cavity or retroperitoneal space comprises inserting, generating, focusing. In an implementation, an optical fiber and ablation device is inserted into the peritoneal or retroperitoneal space via laparoscopic techniques, placing the device adjacent to the material to be ablated using fluoroscopic, fiber-optic, or ultrasound guidance. In an implementation, the light pulses are of less than 100 picoseconds. In an implementation, the light pulses have energies between 10⁻⁷ J and 10⁻² J. In an implementation, the ablation device comprises a focusing optics assembly configured to focus an incoming beam to a peak power density of greater than 10¹² W/cm². In an implementation, an output of the ablation device onto the target tissue or foreign body.

In an implementation, a method of using a femtosecond laser ablation device to ablate biological tissue or a foreign body within the urethra, bladder, ureters, or renal calyces comprises inserting, generating, focusing. In an implementation, an optical fiber and ablation device is inserted and guided via cystoscopy or direct puncture of the renal calyces, placing the device adjacent to the material to be ablated using fluoroscopic, fiber-optic, or ultrasound guidance. In an implementation, the light pulses are of less than 100 picoseconds. In an implementation, the light pulses have energies between 10⁻⁷ J and 10⁻² J. In an implementation, the ablation device comprises a focusing optics assembly configured to focus an incoming beam to a peak power density of greater than 10¹² W/cm². In an implementation, an output of the ablation device is focused onto the target tissue or foreign body.

In an implementation of any of the above use cases, the housing of the device may also incorporate a second lumen to allow passage of an intravascular ultrasound probe, to facilitate guidance and positioning of the ablation tip.

In an implementation, the femtosecond laser ablation device incorporates an additional lumen for passage of a guidewire along the length of the device in an over the wire configuration to facilitate navigation and advancement of the ablation device through the vasculature.

In an implementation, the femtosecond laser ablation device incorporates an additional lumen in the distal portion of the endcap / fiber for passage of a guidewire in a rapid exchange configuration to facilitate navigation and advancement of the ablation device through the vasculature.

In an implementation, the ablation tip may be positioned in the sub-intimal space of a chronic occlusion, adjacent to the true lumen, and the subintimal tissue may be ablated to allow creation of a channel into the distal true lumen of an occluded vessel.

## Claims

1. A system comprising:
an optical fiber (6) configured to transmit light pulses from a laser (1) configured to output sub-100ps light pulses;
an optical fiber endcap device (9) comprising one or more optical elements configured to lower and then raise the intensity of the light pulses from the optical fiber (6) and create an optical pattern from the light pulses of the optical fiber (6);
wherein the one or more optical elements are configured to focus the light pulses from the optical fiber (6) to a peak power density of greater than 10¹² W/cm².

2. The system of claim 1, wherein the optical fiber endcap device (9) is less than 7 mm in diameter.

3. The system of claim 1, wherein the optical pattern is a line, spiral or point cloud.

4. The system of claim 1, wherein the one or more optical elements comprise a spacer (23) configured to allow divergence of a beam outputted by the optical fiber (6) to a peak power density below an optical damage threshold prior to focusing by the one or more optical elements.

5. The system of claim 1, wherein the optical fiber endcap device (9) further comprises an actuator (15) configured to rotate or translate a final ablation pattern by rotating the one or more optical elements or the optical fiber endcap device (9) around a central axis.

6. The system of claim 1, wherein the one or more optical elements comprise:
a first lens (24) configured to focus an incoming beam to a line in a first direction; and
a second lens (25) configured to expand the incoming beam focused by the first lens in a second direction perpendicular to the first direction.

7. The system of claim 1, wherein the one or more optical elements comprise:
a transparent optical cylinder (28); and
a mirror (29) diagonally intersecting the transparent optical cylinder (28) and configured to reflect an incoming beam in a perpendicular direction through the transparent optical cylinder (28).

8. The system of claim 1, wherein the one or more optical elements comprise:
a transparent optical cylinder (28);
a beam splitter configured to reflect a portion of an incoming beam through the transparent optical cylinder (28); and
one or more lenses configured to focus and expand a non-reflected portion of the incoming beam into a forward-facing light sheet.

9. The system of claim 1, wherein the one or more optical elements further comprise:
a transparent optical cylinder (28); and
an electrically switchable transflective mirror configured to switch between reflecting an incoming beam through the transparent optical cylinder and focusing the incoming beam through one or more lenses configured to focus the incoming beam into a forward-facing light sheet.

10. The system of claim 1, wherein the one or more optical elements are embedded in a side of a flexible hollow shell comprising a central cavity configured to capture foreign bodies.

## Patentansprüche

1. System, umfassend:
eine optische Faser (6), die dazu konfiguriert ist, Lichtimpulse von einem Laser (1) zu übertragen, der dazu konfiguriert ist, Lichtimpulse unter 100 ps auszugeben;
eine Endkappenvorrichtung (9) für optische Fasern, die ein oder mehrere optische Elemente umfasst, die dazu konfiguriert sind, die Intensität der Lichtimpulse aus der optischen Faser (6) zu senken und dann anzuheben und aus den Lichtimpulsen der optischen Faser (6) ein optisches Muster zu erzeugen;
wobei das eine oder die mehreren optischen Elemente dazu konfiguriert sind, die Lichtimpulse aus der optischen Faser (6) auf eine Spitzenleistungsdichte von mehr als 10¹² W/cm² zu fokussieren.

2. System nach Anspruch 1, wobei die Endkappenvorrichtung (9) für optische Fasern einen Durchmesser von weniger als 7 mm aufweist.

3. System nach Anspruch 1, wobei das optische Muster eine Linie, eine Spirale oder eine Punktwolke ist.

4. System nach Anspruch 1, wobei das eine oder die mehreren optischen Elemente einen Abstandshalter (23) umfassen, der dazu konfiguriert ist, eine Divergenz eines von der optischen Faser (6) ausgegebenen Strahls auf eine Spitzenleistungsdichte unterhalb einer optischen Schadensschwelle vor dem Fokussieren durch das eine oder die mehreren optischen Elemente zulässt.

5. System nach Anspruch 1, wobei die Endkappenvorrichtung (9) für optische Fasern ferner ein Stellglied (15) umfasst, das dazu konfiguriert ist, ein endgültiges Abtragungsmuster durch Drehen des einen oder der mehreren optischen Elemente oder der Endkappenvorrichtung (9) für optische Fasern um eine Mittelachse zu drehen oder zu verschieben.

6. System nach Anspruch 1, wobei das eine oder die mehreren optischen Elemente Folgendes umfassen:
eine erste Linse (24), die dazu konfiguriert ist, einen einfallenden Strahl auf eine Linie in einer ersten Richtung zu fokussieren; und
eine zweite Linse (25), die dazu konfiguriert ist, den von der ersten Linse fokussierten einfallenden Strahl in einer zweiten Richtung senkrecht zur ersten Richtung zu erweitern.

7. System nach Anspruch 1, wobei das eine oder die mehreren optischen Elemente Folgendes umfassen:
einen transparenten optischen Zylinder (28); und
einen Spiegel (29), der den transparenten optischen Zylinder (28) diagonal schneidet und dazu konfiguriert ist, einen einfallenden Strahl in einer senkrechten Richtung durch den transparenten optischen Zylinder (28) zu reflektieren.

8. System nach Anspruch 1, wobei das eine oder die mehreren optischen Elemente Folgendes umfassen:
einen transparenten optischen Zylinder (28);
einen Strahlteiler, der dazu konfiguriert ist, einen Abschnitt eines einfallenden Strahls durch den transparenten optischen Zylinder (28) zu reflektieren; und
eine oder mehrere Linsen, die dazu konfiguriert sind, einen nicht reflektierten Abschnitt des einfallenden Strahls zu fokussieren und zu einem nach vorne gerichteten Lichtblatt zu erweitern.

9. System nach Anspruch 1, wobei das eine oder die mehreren optischen Elemente ferner Folgendes umfassen:
einen transparenten optischen Zylinder (28); und
einen elektrisch schaltbaren transflektiven Spiegel, der dazu konfiguriert ist, zwischen dem Reflektieren eines einfallenden Strahls durch den transparenten optischen Zylinder und dem Fokussieren des einfallenden Strahls durch eine oder mehrere Linsen, die dazu konfiguriert sind, den einfallenden Strahl in ein nach vorne gerichtetes Lichtblatt zu fokussieren, umzuschalten.

10. System nach Anspruch 1, wobei das eine oder die mehreren optischen Elemente in eine Seite einer flexiblen Hohlschale eingebettet sind, die einen zentralen Hohlraum umfasst, der dazu konfiguriert ist, Fremdkörper aufzufangen.

## Revendications

1. Système comprenant :
une fibre optique (6) configurée pour transmettre des impulsions lumineuses à partir d'un laser (1) configuré pour émettre des impulsions lumineuses inférieures à 100 ps ;
un dispositif d'embout de fibre optique (9) comprenant un ou plusieurs éléments optiques configurés pour réduire puis augmenter l'intensité des impulsions lumineuses provenant de la fibre optique (6) et créer un motif optique à partir des impulsions lumineuses de la fibre optique (6) ;
dans lequel l'un ou les plusierus éléments optiques sont configurés pour focaliser les impulsions lumineuses provenant de la fibre optique (6) à une densité de puissance de crête supérieure à 10¹² W/cm².

2. Système selon la revendication 1, dans lequel le dispositif d'embout de fibre optique (9) a un diamètre inférieur à 7 mm.

3. Système selon la revendication 1, dans lequel le motif optique est une ligne, une spirale ou un nuage de points.

4. Système selon la revendication 1, dans lequel l'un ou les plusieurs éléments optiques comprennent un espaceur (23) configuré pour permettre la divergence d'un faisceau émis par la fibre optique (6) à une densité de puissance de crête supérieure en dessous d'un seuil de dommage optique avant la focalisation par l'un ou les plusieurs éléments optiques.

5. Système selon la revendication 1, dans lequel le dispositif d'embout de fibre optique (9) comprend en outre un actionneur (15) configuré pour faire tourner ou translater un motif d'ablation final par rotation de l'un ou les plusieurs éléments optiques ou du dispositif d'embout de fibre optique (9) autour d'un axe central.

6. Système selon la revendication 1, dans lequel l'un ou les plusieurs éléments optiques comprennent :
une première lentille (24) configurée pour focaliser un faisceau entrant sur une ligne dans une première direction ; et
une deuxième lentille (25) configurée pour étendre le faisceau entrant focalisé par la première lentille dans une deuxième direction perpendiculaire à la première direction.

7. Système selon la revendication 1, dans lequel l'un ou les plusieurs éléments optiques comprennent :
un cylindre optique transparent (28) ; et
un miroir (29) croisant en diagonale le cylindre optique transparent (28) et configuré pour réfléchir un faisceau entrant dans une direction perpendiculaire à travers le cylindre optique transparent (28).

8. Système selon la revendication 1, dans lequel l'un ou les plusieurs éléments optiques comprennent :
un cylindre optique transparent (28) ;
un diviseur de faisceau configuré pour réfléchir une partie d'un faisceau entrant à travers le cylindre optique transparent (28) ; et
une ou plusieurs lentilles configurées pour focaliser et étendre une partie non réfléchie du faisceau entrant dans une feuille de lumière orientée vers l'avant.

9. Système selon la revendication 1, dans lequel l'un ou les plusieurs éléments optiques comprennent en outre :
un cylindre optique transparent (28) ; et
un miroir transflectif commutable électriquement configuré pour commuter entre la réflexion d'un faisceau entrant à travers le cylindre optique transparent et la focalisation du faisceau entrant à travers une ou plusieurs lentilles configurées pour focaliser le faisceau entrant dans une feuille de lumière orientée vers l'avant.

10. Système selon la revendication 1, dans lequel l'un ou les plusieurs éléments optiques sont intégrés dans un côté d'une coque creuse flexible comprenant une cavité centrale configurée pour capturer des corps étrangers.
